# EUROPÄISCHE PATENTSCHRIFT

(11) **EP 0 659 062 B1**
(45) Veröffentlichungstag und Bekanntmachung des Hinweises auf die Patenterteilung: **27.05.2009**
(21) Anmeldenummer: 93912539.9
(22) Anmeldetag: 02.07.1993
(51) Int. Cl.: A61B 17/60, A61B 17/70

(54) **Posteriores Wirbelsäulenimplantat**
Posterior vertebral column implant
Implant vertébral postérieur

(43) Veröffentlichungstag der Anmeldung: 28.06.1995
(73) Patentinhaber: Synthes GmbH, 4436 Oberdorf (CH)
(72) Erfinder: SCHLÄPFER, Johannes, Fridolin, CH-8750 Glarus (CH); HESS, Martin, CH-4434 Hölstein (CH); SCHELKER, René, CH-4431 Bennwil (CH); HAGMANN, Ralf, CH-4431 Bennwil (CH)
(74) Vertreter: Lusuardi, Werther
(86) Internationale Anmeldenummer: PCT/CH1993/000170
(87) Internationale Veröffentlichungsnummer: WO 1995/001132

(56) Entgegenhaltungen:
- EP-A1- 0 441 729
- EP-A2- 0 465 158
- DE-A- 4 110 002
- DE-A1- 3 800 052

## Beschreibung

Die vorliegende Erfindung betrifft ein posteriores Wirbelsäulenimplantat gemäss dem Oberbegriff des Patentanspruchs 1.

Solche Implantate dienen zur Korrektur von sagittalen Wirbelsäulendeformitäten (Kyphose, Spondylolisthesis) oder zur Stabilisierung ohne Korrektur (degenerative Instabilitäten). Sie weisen typischerweise einen Befestigungskopf, einen mit ihm verbundenen Verankerungsabschnitt und eine Verschlussschraube auf, die auf einen in einem Schlitz des Befestigungskopfes aufgenommenen Stützstab drückt. Weiter kann auf dem Befestigungskopf eine Stabilisierungsstütze aufgesetzt sein.

Der Verankerungsabschnitt kann als Schraube oder Haken ausgebildet sein, wobei die Schraube direkt über dem Pedikel im Wirbelkörper befestigt wird, der Haken hingegen am Wirbel eingehängt wird. Zur Wirbelsäulenkorrektur oder - stabilisierung sind entsprechend der Anzahl zu korrigierender oder stabilisierender Wirbel und dem gewählten Mittel mehrere Schrauben oder Haken erforderlich, die mit dem Stützstab fest verbunden und damit fixiert sind. Bei diesen bekannten Wirbelsäulenimplantaten wird die Verschlussschraube meist direkt in den Befestigungskopf eingedreht, nachdem der Stützstab in den Schlitz eingeführt worden ist. Dabei treten folgende Nachteile auf:

An seinem Ende besteht der Befestigungskopf durch den U-förmigen Schlitz zur Aufnahme des Stützstabes bedingt nur aus zwei tulpenförmigen Backen, so dass die Verschlussschraube nur im Gewinde dieser Backen aufliegt. Falls die Verschlussschraube unter Druck eingedreht werden muss, hat der Operateur Schwierigkeiten, zu fühlen, ob die Schraube richtig ins Gewinde eingeführt worden ist. Weiter muss zur Verhinderung eines Aufweitens des Schlitzes und damit des Gewindes eine Stabilisierungsstütze aufgesetzt werden.

Es ist auch bereits ein Implantat aus der FR-A 2 624 720 bekannt, bei welchem die beiden einen U-förmigen Schlitz bildenden Schenkel des Befestigungskopfes ein Aussengewinde aufweisen, auf welches eine Verschlusskappe aufschraubbar ist, durch deren zentrale Gewindebohrung das eigentliche Fixierteil einschraubbar ist. Diese bekannte Vorrichtung ist jedoch fertigungsmässig nicht befriedigend und führt zudem zu einer unerwünschten Vergrösserung des Befestigungskopfes in Höhe und Durchmesser.
Schliesslich ist aus der EP-A-0 465 158 MEHDIAN ein Implantat bekannt, bei welchem eine Klemmschraube in das unvollständige Gewinde des U-förmigen Befestigungskopfes eingeschraubt werden kann, um einen im U-förmigen Schlitz liegenden Stützstab zu klemmen. Zusätzlich zu dieser zwingend vorgesehenen Erstfixierung des Stützstabes wird bei dieser bekannten Vorrichtung weiter vorgeschlagen, durch die Klemmschraube hindurch, eine zusätzliche Fixationsschraube in Anlage zum Stützstab zu bringen.

Die vorliegende Erfindung stellt sich nun die Aufgabe diese Nachteile zur vermeiden. Diese Aufgabe wird durch ein posteriores Wirbelsäulenimplantat gelöst, das die kennzeichnenden Merkmale des Patentanspruches 1 aufweist. Weitere vorteilhafte Merkmale sind in den abhängigen Patentansprüchen beschrieben.

Der wesentliche Vorteil der Erfindung ist, dass das Fixierteil in ein geschlossenes Gewinde einführbar ist. Bei einem offenen, geschlitzten Gewinde entsteht aufgrund der Inkongruenz zwischen dem Innen- und Aussengewinde eine grosse Reibung beim Anziehen des Fixier-Teils. Die Reibung kann je nach Situation so gross sein, dass ein Kaltverschweissen zwischen Fixier-Teil und Befestigungskopf auftritt.

Ein weiterer Vorteil ist, dass das Volumen des Befestigungskopfes durch die Fixationsmittel nicht vergrössert wird, so dass das Implantat besser verträglich ist.

Einige Ausführungsbeispiele erfindungsgemässer posteriorer Wirbelsäulenimplantate werden anhand der Zeichnung näher erläutert. Dabei werden als Verankerungsabschnitte bloss Schraubenschäfte dargestellt; letztere können aber auch durch Haken ersetzt werden.

Es zeigen:
Fig. 1 in einer auseinandergezogenen, perspektivischen Darstellungsweise ein erfindungsgemässes Wirbelsäulenimplantat;
Fig. 2 - 5 in einer auseinandergezogenen, perspektivischen Darstellungsweise weitere Ausführungsformen von erfindungsgemässen Wirbelsäulenimplantaten;
Fig. 6 einen Längsschnitt durch den Befestigungskopf des Wirbelsäulenimplantates; und
Fig. 7 eine Längsschnitt durch den Verschluss- und Fixier-Teil des Wirbelsäulenimplantates.

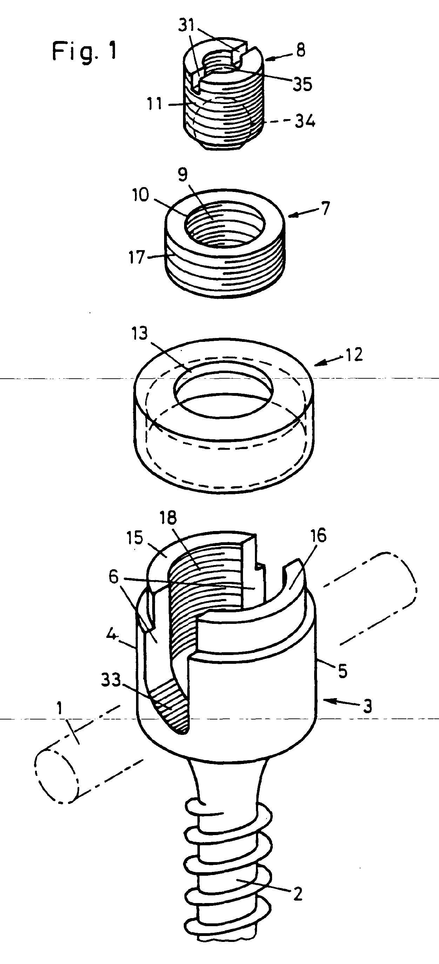

Beim in der Fig. 1 dargestellten Wirbelsäulenimplantat bilden Befestigungskopf 3 und Verankerungsabschnitt 2 eine Pedikelschraube, welche in einen Wirbel einschraubbar ist. Zur Aufnahme eines Stützstabes 1 weist der Befestigungskopf 3 einen durch zwei Schenkel 4,5 gebildeten U-förmigen Schlitz 6 auf. Zur besseren Haftung des Stützstabes 1 am Schlitzgrund ist letzterer mit einer Längsverzahnung 33 versehen. Weiter ist im oberen Bereich des Befestigungskopfes 3 ein Innengewinde 18 vorgesehen. Ein in den U-förmigen Schlitz 6 des Befestigungskopfes 3 eindrehbares zylindrisches Verschluss-Teil 7 weist ein mit dem Innengewinde 18 korrespondierendes Aussengewinde 17 auf, sowie eine mit einem Innengewinde 9 versehene zentrale Bohrung 10.

Ein Fixier-Teil 8 mit einem zum Innengewinde 9 korrespondierenden Aussengewinde 11 ist in die Bohrung 10 einschraubbar. Das Fixier-Teil 8 weist zur besseren Manipulierbarkeit oben einen Schlitz 31 auf. Unten ist ein bewegliches Kugelsegment (34) eingepresst.
Schliesslich ist eine Sicherheitshülse 12 vorgesehen, welche oben einen Bund 13 aufweist. Die Sicherheitshülse 12 wird über die nach oben verjüngten Schenkelenden 15,16 des Befestigungskopfes 3 geschoben. Die Sicherheitshülse 12 kann auch einstückig mit dem Verschluss-Teil 7 verbunden sein.

Der Durchmesser des Gewindes 18 im Befestigungskopf 3 und des Gewindes 17 im Verschluss-Teil 7 liegt zweckmässigerweise im Bereich von 12 - 14 mm, vorzugsweise von 12,5 - 13,5 mm. Der Durchmesser des Fixier-Teils 8 liegt im Bereich von 7 - 9 mm, vorzugsweise von 7,5 - 8,5 mm. Die Breite des U-förmigen Schlitzes 6 im Befestigungskopfes 3 und der Durchmesser des Stützstabes 1 liegt im Bereich von 5 - 7 mm, vorzugsweise von 5,5, - 6,5 mm.

Bei einer Operation wird die Pedikelschraube mit dem Verankerungsabschnitt 2 in den zu korrigierenden oder stabilisierenden Wirbel eingeschraubt und der Stützstab 1 in den U-förmigen Schlitz 6 des Befestigungskopfes eingelegt. Darauf wird das Verschluss-Teil 7 axial von oben in den U-förmigen Schlitz 6 eingedreht bis der obere Rand bündig mit den beiden Schenkeln 15,16 ist. Das Fixier-Teil 8 kann dabei bereits geringfügig im Verschluss-Teil 7 eingeschraubt sein. Nach der bisherigen klinischen Erfahrung sind die Schrauben in den verschiedenen Wirbeln immer derart angeordnet, dass genügend Raum zum axialen Einschrauben des Verschluss-Teils 7 vorhanden ist. Dies ist auch der Fall, wenn Haken verwendet werden.
Wenn die Sicherheitshülse 12 nicht fest mit dem Verschluss-Teil 7 verbunden ist, muss sie jetzt aufgesetzt werden, bevor das Fixier-Teil 8 angezogen ist. Sie verhindert ein Aufspreizen der Schenkel 4,5.
Anschliessend wird das Fixier-Teil 8 mit einem mit seinem Schlitz 31 vorübergehend verbindbaren (zeichnerisch nicht dargestellten) Verlängerungsstück in den Verschluss-Teil 7 so weit eingeschraubt, bis das Kugelsegment 34 auf dem Stützstab 1 aufliegt, und dann angezogen. Dadurch wird der Stützstab 1 im U-förmigen Schlitz 6 festgeklemmt. Das Kugelsegment 34 ist derart konstruiert, dass bei einer Belastung in der Längsrichtung des Stützstabes 1 die Klemmkraft proportional zunimmt. Das Verlängerungsstück wird nach dem Klemmprozess vom Fixier-Teil 8 gelöst, der nun ein wenig im Verschluss-Teil 7 versenkt ist. Der gleiche Vorgang wiederholt sich bei den anderen Implantaten.

Das beschriebene Wirbelsäulenimplantat weist zahlreiche Vorteile auf. So ist das Fixier-Teil 8, mit dem der Stützstab 1 geklemmt wird, in einem geschlossenen Gewinde geführt und wird erst eingedreht, wenn der Stützstab 1 richtig positioniert worden ist. Im Gegensatz zu den herkömmlichen Implantaten mit posteriorer Öffnung dient das geschlitzte Gewinde 18 im Befestigungskopf 3 nicht zum Spannen sondern nur zum Fixieren des Verschluss-Teils 7. Das Fixier-Teil 8 kann schon vor dem Einschrauben des Verschluss-Teils 7 ein wenig in diesen eingedreht werden, so dass das Verschluss-Teil leicht manipulierbar wird. Dadurch dass der Aussendurchmesser der Fixationsmittel 7,8 kleiner oder maximal gleich gross wie der Aussendurchmesser des Befestigungskopfes 3 ist, ergibt sich keine Vergrösserung des Implantates im Durchmesser.

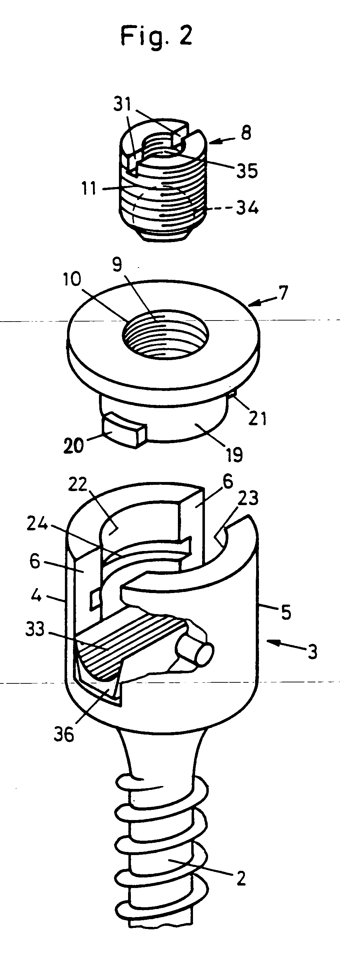

In den Fig. 2 - 5 wird das Verschluss-Teil 7 nicht durch Verschraubung wie in Fig. 1 sondern bajonettartig mit dem Befestigungskopf 3 verbunden. Wie schon in Fig. 1 gezeigt, wird das Fixier-Teil 8 in ein komplettes, im Verschluss-Teil 7 befindliches Innengewinde 9 eingedreht.

Bei dem in Fig. 2 dargestellten modifizierten Wirbelsäulenimplantat weist der Verschluss-Teil 7 an seiner Mantelfläche 19 zwei gegenüberliegende Nocken 20,21 auf. Analog dazu weisen die beiden Schenkel 4,5 des U-förmigen Schlitzes 6 des Befestigungskopfes 3 an ihren Innenseiten 22,23 damit korrespondierende zirkuläre Nuten 24,25 auf. Nach der axialen Einführung wird das Verschluss-Teil 7 im U-förmigen Schlitz 6 gedreht, wobei die Nocken 20 und 21 in die Nuten 24,25 eingeführt werden.
Bei dieser Ausführungsform kann das Fixier-Teil 8 unten ebenfalls ein rotierbares, kugelförmiges Kontaktelement in Form eines Kugelsegmentes 34 aufweisen, um eine bessere Anpassung an die Oberflächengeometrie des Stützstabes 1 zu erzielen. Die Einzelheiten dieses Kontaktelementes 34 sind in der noch unveröffentlichten europ. Patentanmeldung Nr. 93106520.5 beschrieben.

Der Boden des U-förmigen Schlitzes 6 weist in dessen Längsrichtung eine konkave Ausnahme auf, in welche ein bewegliches Segment 36 mit Längsverzahnung 33 eingelegt ist, wobei die Bewegung des Segmentes 36 durch die Geometrie der konkaven Ausnahme gegeben ist.

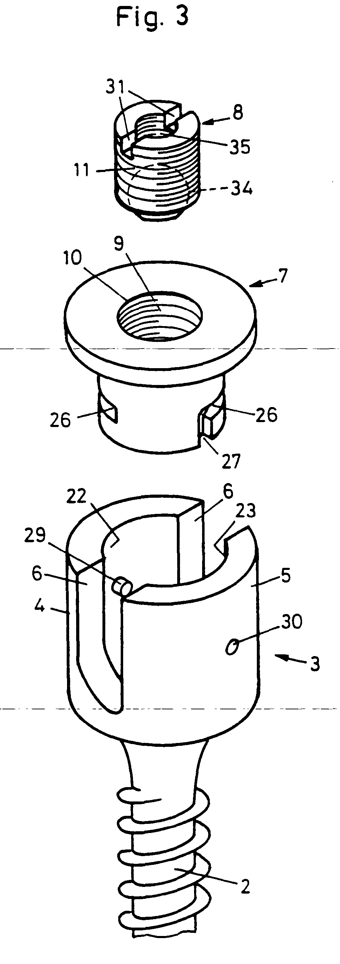

In Fig. 3 weist das Verschluss-Teil 7 an seiner Mantelfläche 19 eine zirkuläre Quernut 26 und zwei gegenüberliegende Längsnuten 27,28 auf. Die Längsnuten 27,28 stimmen dabei mit auf den Innenseiten 22,23 der beiden Schenkel 4,5 befindlichen Nocken 29,30 in Position und Richtung überein. Nach der axialen Einführung des Verschluss-Teils 7 in den U-förmigen Schlitz 6 sind die Nocken 29,30 durch eine Drehbewegung in die zirkuläre Quernut 26 einführbar. Vorzugsweise nimmt die zirkuläre Quernut 26 nicht den gesamten Umfang des Verschluss-Teils 7 ein.

Bei den in Fig. 2 und 3 gezeigten Ausführungsformen ist eine Sicherheitshülse nicht unbedingt notwendig, da die beiden Schenkel 4,5 aufgrund des bajonettartigen Verschlusses beim Anziehen des Fixier-Teils 8 nicht die Tendenz haben, aufzuspreizen

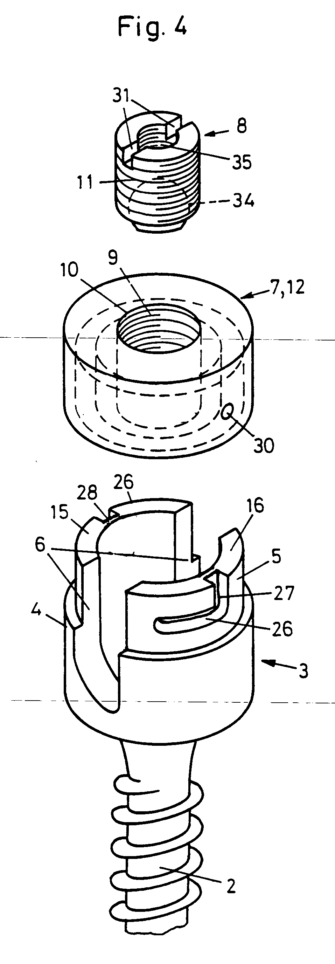

In Fig. 4 ist eine Ausführungsform dargestellt, bei welcher das Verschluss-Teil 7 und die Sicherheitshülse 12 einstückig miteinander verbunden sind. Die Blockierung des Verschluss-Teil im Befestigungskopf 3 erfolgt ähnlich zu den Ausführungsformen gemäss Fig. 2 und 3. Die zirkuläre Quernut 26 mit Längsnuten 27,28 ist hier - statt auf der Innenfläche der Schenkel 4,5 - auf der Aussenfläche der nach oben verjüngten Schenkelenden 15,16 des Befestigungskopfes 3 angebracht. Die Längsnuten 27,28 stimmen dabei mit im Innern der Sicherheitshülse 12 befindlichen Nocken 29,30 in Position und Richtung überein. Nach der axialen Einführung des Verschluss-Teils 7 in den U-förmigen Schlitz 6 sind die Nocken 29,30 durch eine Drehbewegung in die zirkuläre Quernut 26 einführbar.

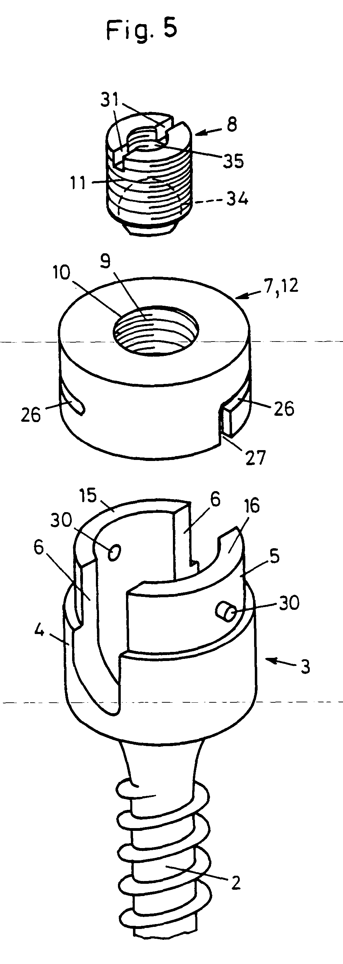

In Fig. 5 ist eine zur Fig. 4 reziproke Ausführungsform dargestellt, bei welcher zirkuläre Quernut 26 mit Längsnuten 27,28 in der Sicherheitshülse 12 untergebracht sind und die Nocken 29,30 auf der Aussenfläche der nach oben verjüngten Schenkelenden 15,16 des Befestigungskopfes 3.

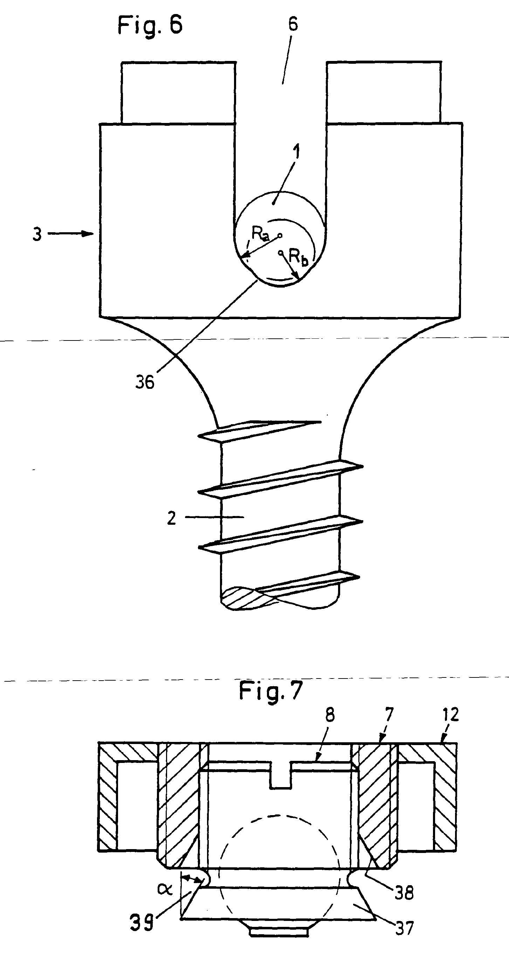

In Fig. 6 ist dargestellt, wie der Grund des U-förmigen Schlitzes 6 des Befestigungskopfes 3 mit einem Doppelradius Ra,Rb versehen ist, um die Aufnahme von Stützstäben 1 mit unterschiedlichem Durchmesser aufnehmen zu können.

In Fig. 7 ist schliesslich eine bevorzugte Ausführungsform des Fixier-Teils 7 dargestellt, welches zum Stützstab 1 hin einen Flansch 37 aufweist. Der Flansch 37 weist auf der vom Stützstab 1 abgewandten Seite einen Winkel 39 zwischen α = 0° - 90°, vorzugsweise zwischen 45° - 60° auf. Entsprechend dazu weist das Verschluss-Teil 8 eine Ansenkung 38 auf.
Der Flansch 37 und die Ansenkung 38 haben den Zweck, das Verschluss-Teil 7 und das Fixier-Teil 8 vor deren Montage so stark gegeneinander zu verspannen, dass sich das Verschluss-Teil 7 und das Fixier-Teil 8 bis zu einem bestimmten Anziehmoment als Einheit verhalten und erst beim Überschreiten dieses Anziehmomentes voneinander separieren. Das Separieren geschieht dadurch, dass das Verschluss-Teil 7 oder die Stabilisierhülse 12 beim Eindrehen plötzlich ansteht und nur noch das Fixier-Teil 8 weiter eindringen kann, bis es auf dem Stützstab 1 aufsteht und beginnt diesen zu klemmen.

## Patentansprüche

1. Posteriores Wirbelsäulenimplantat für eine, einen Stützstab (1) aufweisende Vorrichtung zur Abstützung der Wirbelsäule, mit
A) einem als Schraube oder Haken ausgebildeten Verankerungsabschnitt (2) und einem Befestigungskopf (3), der einen, zwei Schenkel (4,5) bildendenden, U-förmigen Schlitz (6) für den Stützstab (1) aufweist; und mit
B) Fixationsmitteln (7,8) zur Fixierung des Befestigungskopfes (3) am Stützstab (1), wobei die Fixationsmittel (7,8) zweiteilig ausgebildet sind und ein zylindrisches Verschluss-Teil (7) und eine Fixierteil (8) aufweisen; wobei
C) das zylindrische Verschluss-Teil (7) axial von oben in den U-förmigen Schlitz (6) einführbar ist; und
D) das Fixier-Teil (8) durch das erste Verschluss-Teil (7) hindurch in Anlage zum Stützstab (1) bringbar ist.
**dadurch gekennzeichnet, dass**
E) das zylindrische Verschluss-Teil (7) und der U-förmige Schlitz (6), derart ausgestaltet sind, dass das zylindrische Verschluss-Teil (7) ohne eine Klemmung des kreiszylindrischen Stützstabes (1) zu bewirken, im U-förmigen Schlitz (6) blockierbar ist, so dass einzig das Fixier-Teil (8) in Anlage zum Stützstab (1) bringbar ist.

2. Posteriores Wirbelsäulenimplantat nach Anspruch 1, **dadurch gekennzeichnet, dass** das Verschluss-Teil (7), eine zentrale mit einem Innengewinde (9) versehene Bohrung (10) aufweist, durch welche das Fixier-Teil (8) einführbar ist.

3. Posteriores Wirbelsäulenimplantat nach Anspruch 1, **dadurch gekennzeichnet, dass** das Fixierteil (8) ein mit dem Innengewinde (9) des Verschluss-Teils (7) korrespondierendes Aussengewinde (11) aufweist.

4. Posteriores Wirbelsäulenimplantat nach einem der Ansprüche 1 bis 3, **dadurch gekennzeichnet, dass** der Aussendurchmesser der Fixationsmittel (7,8) kleiner oder maximal gleich gross wie der Aussendurchmesser des Befestigungskopfes (3) ist.

5. Posteriores Wirbelsäulenimplantat nach einem der Ansprüche 1 bis 4, **dadurch gekennzeichnet, dass** eine die beiden Schenkel (4,5) des Befestigungskopfes (3) überkappende Sicherheitshülse (12) vorgesehen ist.

6. Posteriores Wirbelsäulenimplantat nach Anspruch 5, **dadurch gekennzeichnet, dass** die Sicherheitshülse (12) einen so grossen Innendurchmesser aufweist, dass sie über die nach oben verjüngten Schenkelenden (15,16) des Befestigungskopfes (3) geschoben werden kann.

7. Posteriores Wirbelsäulenimplantat nach Anspruch 6, **dadurch gekennzeichnet, dass** die Sicherheitshülse (12) oben einen Bund (13) mit einem so grossen Innendurchmesser aufweist, dass das Fixier-Teil (8) hindurch geführt werden kann.

8. Posteriores Wirbelsäulenimplantat nach Anspruch 6, **dadurch gekennzeichnet, dass** die Sicherheitshülse (12) oben einen Boden mit einer Bohrung aufweist, die so gross ist, dass das darunterliegende Fixier-Teil (8) mit einem entsprechenden Instrument manipuliert werden kann.

9. Posteriores Wirbelsäulenimplantat nach einem der Ansprüche 5 bis 8, **dadurch gekennzeichnet, dass** die Sicherheitshülse (12) und das Verschluss-Teil (7) zu einem Stück gefügt oder als ein Stück gefertigt sind.

10. Posteriores Wirbelsäulenimplantat nach einem der Ansprüche 1 bis 9, **dadurch gekennzeichnet, dass** das Verschluss-Teil (7) ein Aussengewinde (17) aufweist und der U-förmige Schlitz (6) ein damit korrespondierendes Innengewinde (18) aufweist.

11. Posteriores Wirbelsäulenimplantat nach einem der Ansprüche 1 bis 9, **dadurch gekennzeichnet, dass** das Verschluss-Teil (7) an seiner Mantelfläche (19) mindestens einen Nocken (20,21) aufweist und die beiden Schenkel (4,5) des U-förmigen Schlitzes (6) an ihren Innenseiten (22,23) damit korrespondierende zirkuläre Nuten (24,25) aufweisen, derart dass nach der axialen Einführung des Verschluss-Teiles (7) in den U-förmigen Schlitz (6) ein bajonettartiger Verschluss (20,21;24,25) resultiert.

12. Posteriores Wirbelsäulenimplantat nach Anspruch 10, **dadurch gekennzeichnet, dass** das Verschluss-Teil (7) an seiner Mantelfläche (19) zwei gegenüberliegende Nocken (20,21) aufweist.

13. Posteriores Wirbelsäulenimplantat nach einem der Ansprüche 1 bis 9, **dadurch gekennzeichnet, dass** das Verschluss-Teil (7) an seiner Mantelfläche (19) eine zirkuläre Quernut (26) und der Anzahl und Position entsprechende Längsnuten (27,28) aufweist, wobei
a) die Längsnuten (27,28) mit auf den Innenseiten (22,23) der beiden Schenkel (4,5) befindlichen Nocken (29,30) in Position und Richtung übereinstimmen; und
b) nach der axialen Einführung des Verschluss-Teils (7) in den U-förmigen Schlitz (6) die Nocken (29,30) durch eine Drehbewegung in die zirkuläre Quernut (26) einführbar sind.

14. Posteriores Wirbelsäulenimplantat nach Anspruch 13, **dadurch gekennzeichnet, dass** das Verschluss-Teil (7) an seiner Mantelfläche (19) eine zirkuläre Quernut (26) und zwei gegenüberliegende Längsnuten (27,28) aufweist.

15. Posteriores Wirbelsäulenimplantat nach Anspruch 11 bis 14, **dadurch gekennzeichnet, dass** die zirkuläre Quernut (24;26) nicht den gesamten Umfang des Befestigungskopfes (3), bzw. des Verschluss-Teils (7) einnimmt.

16. Posteriores Wirbelsäulenimplantat nach einem der Ansprüche 1 bis 15, **dadurch gekennzeichnet, dass** der Fixier-Teil (8) als Madenschraube ausgebildet ist.

17. Posteriores Wirbelsäulenimplantat nach Anspruch 16, **dadurch gekennzeichnet, dass** die Madenschraube oben einen Schlitz (31) und eine zentrische Bohrung (35) aufweist.

18. Posteriores Wirbelsäulenimplantat nach Anspruch 17, **dadurch gekennzeichnet, dass** die zentrische Bohrung (35) mit einem Gewinde versehen ist.

19. Posteriores Wirbelsäulenimplantat nach einem der Ansprüche 16 bis 18, **dadurch gekennzeichnet, dass** die Madenschraube zum Stützstab (1) hin eine konvexe Oberfläche (32) aufweist.

20. Posteriores Wirbelsäulenimplantat nach Anspruch 19, **dadurch gekennzeichnet, dass** die Madenschraube zum Stützstab (1) hin eine bombierte Oberfläche (32) aufweist.

21. Posteriores Wirbelsäulenimplantat nach Anspruch 20, **dadurch gekennzeichnet, dass** die bombierte Oberfläche (32) mit einer Einsenkung versehen ist.

22. Posteriores Wirbelsäulenimplantat nach Anspruch 21, **dadurch gekennzeichnet, dass** die Einsenkung kegelförmig ausgebildet ist.

23. Posteriores Wirbelsäulenimplantat nach einem der Ansprüche 16 bis 22, **dadurch gekennzeichnet** die Madenschraube unten in eine Spitze ausläuft.

24. Posteriores Wirbelsäulenimplantat nach einem der Ansprüche 1 bis 23, **dadurch gekennzeichnet, dass** das Fixier-Teil (8) unten mit einem rotierbaren, kugelförmigen Kontaktelement versehen ist.

25. Posteriores Wirbelsäulenimplantat nach Anspruch 24, **dadurch gekennzeichnet, dass** das rotierbare, kugelförmige Kontaktelement in Form eines Kugelsegmentes (34) ausgebildet ist.

26. Posteriores Wirbelsäulenimplantat nach einem der Ansprüche 1 bis 25, **dadurch gekennzeichnet, dass** der Befestigungskopf (3) auf dem Grund des U-förmigen Schlitzes (6) mit Längsverzahnungen (33) versehen ist.

27. Posteriores Wirbelsäulenimplantat nach einem der Ansprüche 1 bis 26, **dadurch gekennzeichnet, dass** der Befestigungskopf (3) auf dem Grund des U-förmigen Schlitzes (6) mit einem Quermuster versehen ist, in welches sich der Stützstab (1) eingraben kann.

28. Posteriores Wirbelsäulenimplantat nach einem der Ansprüche 1 bis 27, **dadurch gekennzeichnet, dass** der Grund des U-förmigen Schlitzes (6) mehr als einen Radius aufweist.

29. Posteriores Wirbelsäulenimplantat nach Anspruch 28, **dadurch gekennzeichnet, dass** der Grund des U-förmigen Schlitzes (6) zwei Radien Rₐ,R_{b} aufweist.

30. Posteriores Wirbelsäulenimplantat nach einem der Ansprüche 1 bis 29, **dadurch gekennzeichnet, dass** der Durchmesser von Befestigungskopf (3) und Verschluss-Teil (7) im Bereich von 12 - 14 mm liegt.

31. Posteriores Wirbelsäulenimplantat nach Anspruch 30, **dadurch gekennzeichnet, dass** der Durchmesser von Befestigungskopf (3) und Verschluss-Teil (7) im Bereich von 12,5 - 13,5 mm liegt.

32. Posteriores Wirbelsäulenimplantat nach einem der Ansprüche 1 bis 31, **dadurch gekennzeichnet, dass** der Durchmesser des Fixier-Teils (8) im Bereich von 7 - 9 mm liegt.

33. Posteriores Wirbelsäulenimplantat nach Anspruch 32, **dadurch gekennzeichnet, dass** der Durchmesser des Fixier-Teils (8) im Bereich von 7,5 - 8,5 mm liegt.

34. Posteriores Wirbelsäulenimplantat nach einem der Ansprüche 1 bis 33, **dadurch gekennzeichnet, dass** die Breite des U-förmigen Schlitzes (6) im Befestigungskopfes (3) und der Durchmesser des Stützstabes (1) im Bereich von 5 - 7 mm liegt.

35. Posteriores Wirbelsäulenimplantat nach einem der Ansprüche 1 bis 34, **dadurch gekennzeichnet, dass** die Breite des U-förmigen Schlitzes (6) im Befestigungskopfes (3) und der Durchmesser des Stützstabes (1) im Bereich von 5,5, - 6,5 mm liegt.

36. Posteriores Wirbelsäulenimplantat nach einem der Ansprüche 1 bis 25 oder 30 bis 35, **dadurch gekennzeichnet, dass** der Boden des U-förmigen Schlitzes (6) in dessen Längsrichtung eine konkave Ausnahme aufweist und ein bewegliches Segment (36) darin eingelegt ist, wobei die Bewegung des Segmentes (36) durch die Geometrie der konkaven Ausnahme gegeben ist.

37. Posteriores Wirbelsäulenimplantat nach Anspruch 36, **dadurch gekennzeichnet, dass** die Bewegung des Segmentes kreisförmig ist, wobei das Drehzentrum identisch zum Zentrum des kugelförmigen Kontaktelementes (34 ) ist.

38. Posteriores Wirbelsäulenimplantat nach Anspruch 36, **dadurch gekennzeichnet, dass** die Bewegung des Segmentes kreisförmig ist, wobei das Drehzentrum an der Stelle auf dem Stützstab (1) liegt, wo die Spitze des Fixier-Teils (8) aufliegt.

39. Posteriores Wirbelsäulenimplantat nach einem der Ansprüche 1 bis 23, **dadurch gekennzeichnet, dass** die Ausnahme im Boden des U-förmigen Schlitzes (6) kugelförmig ist.

40. Posteriores Wirbelsäulenimplantat nach Anspruch 39, **dadurch gekennzeichnet, dass** der Radius der Ausnahme identisch zu einer über den Stützstab (1) geschobenen Kugel ist.

41. Posteriores Wirbelsäulenimplantat nach Anspruch 40, **dadurch gekennzeichnet, dass** die über den Stützstab (1) geschobenen Kugel einseitig geschlitzt ist.

42. Posteriores Wirbelsäulenimplantat nach einem der Ansprüche 1 bis 41, **dadurch gekennzeichnet, dass** das Fixier-Teil (8) zum Stützstab (1) hin einen Flansch (37) aufweist.

43. Posteriores Wirbelsäulenimplantat nach Anspruch 42, **dadurch gekennzeichnet, dass** der Flansch (37) auf der vom Stützstab (1) abgewandten Seite einen Winkel α (39) zwischen 0° und 90° aufweist.

44. Posteriores Wirbelsäulenimplantat nach Anspruch 43, **dadurch gekennzeichnet, dass** der Flansch (37) auf der vom Stützstab (1) abgewandten Seite einen Winkel α (39) zwischen 45° und 60° aufweist.

45. Posteriores Wirbelsäulenimplantat nach einem der Ansprüche 42 bis 44, **dadurch gekennzeichnet, dass** das Verschluss-Teil (7) gegen den Stützstab (1) hin eine der Geometrie des Flansches (37) entsprechende Ansenkung (38) aufweist.

## Claims

1. A posterior intervertebral implant for a device including a support rod (1) to support the vertebral column with
(a) an anchoring segment (2) configured as a screw or hook and a fixation head (3) which comprises a U-shaped slot (6) for the support rod (1) and which forms two legs (4, 5); and with
(b) locking means (7, 8) for securing the fixation head (3) to the support rod (1), whereby the locking means (7, 8) is configured two-piecedly and comprises a cylindrical closure element (7) and a fastening member (8); wherein
(c) the cylindrical closure element (7) is axially insertable into the U-shaped slot (6) from the top; and
(d) the fastening member (8) can be brought in contact with the support rod (1) through the first closure element (7),
**characterized in that**
(e) the cylindrical closure element (7) and the U-shaped slot (6) are configured in such manner that the cylindrical closure element (7) is lockable within said U-shaped slot (6) without causing a clamping of the circular cylindrical support rod (1) so that solely said fastening member (8) can be brought in contact with said support rod (1).

2. Posterior intervertebral implant as claimed in claim 1, wherein the closure element (7) comprises a central bore hole (10) which is provided with an internal thread (9) through which the fastening member (8) is insertable.

3. Posterior intervertebral implant as claimed in claim 1, wherein the fastening member (8) has an external thread (11) matching the internal thread (9) of the closure element (8).

4. Posterior intervertebral implant as claimed any one of claims 1 to 3, wherein the outside diameter of the locking means (7, 8) is smaller or maximally equally large than the outer diameter of the fixation head (3).

5. Posterior intervertebral implant as claimed in any one of claims 1 to 4, wherein a safety bush (12) is provided which spans the two legs (4, 5) of the fixation head (3).

6. Posterior intervertebral implant as claimed claim 5, wherein the safety bush (12) has an inner diameter such that it can be slipped over the upwardly tapering ends (15, 16) of the legs of the fixation head (3).

7. Posterior intervertebral implant as claimed in claim 6, wherein the safety bush (12) comprises a collar (13) on top having an inner diameter large enough to allow passage of the fastening member (8).

8. Posterior intervertebral implant as claimed in claim 6, wherein the safety bush (12) comprises a plate with a bore large enough that the fastening member (8) lying below can be manipulated with a respective instrument.

9. Posterior intervertebral implant as claimed in any one of claims 5 to 8, wherein the safety bush (12) and closure element (7) are joined to one piece or are integrally manufactured.

10. Posterior intervertebral implant as claimed in any one of claims 1 to 9, wherein the closure element (7) has an outer thread (17) and the U-shaped slot (6) has a matching inner thread (18).

11. Posterior intervertebral implant as claimed in any of claims 1 to 9, wherein the closure element (7) comprises at least one stud (20, 21) at its peripheral surface (19) and the two legs (4, 5) of the U-shaped slot (6) have arcuate grooves (24, 25) matching thereto such that after axial insertion of the closure element (7) into the U-shaped slot (6) a bayonet type locking (20, 21, 24, 25) results.

12. Posterior intervertebral implant as claimed in claim 10, wherein the closure element (7) comprises two opposite studs (20, 21) at its peripheral surface (19).

13. Posterior intervertebral implant as claimed in any one of the claims 1 to 9, wherein the closure element (7) comprises an arcuate transverse groove (26) and longitudinal grooves (27, 28) corresponding to the number and position, whereby
a) the longitudinal grooves (27, 28) matching studs (29, 30) located at the inner sides (22, 23) of the two legs (4, 5) with respect to position and direction; and
b) after the axial insertion of the closure element (7) into the U-shaped slot (6) the studs (29, 30) are insertable into the arcuate transverse groove (26) by means of a rotating movement.

14. Posterior intervertebral implant as claimed in claim 13, wherein the closure element (7) comprises at its peripheral surface (19) an arcuate transverse groove (26) and two oppositely arranged longitudinal grooves (27, 28).

15. Posterior intervertebral implant as claimed in any one of claims 11 to 14, wherein the arcuate transverse groove (24, 26) extends over less than the full circumference of the fixation head (3), respectively the closure element (7).

16. Posterior intervertebral implant as claimed in any one of claims 1 to 15, wherein the fastening member (8) is configured as a stud screw.

17. Posterior intervertebral implant as claimed in claim 16, wherein the stud screw is provided with a slot (31) and a centric bore (35) on the top.

18. Posterior intervertebral implant as claimed in claim 17, wherein the centric bore (35) is provided with a thread.

19. Posterior intervertebral implant as claimed in any one of claims 16 to 18, wherein the stud screw comprises a convex surface (32) towards the support rod (1).

20. Posterior intervertebral implant as claimed in claim 19, wherein the stud screw comprises a embossed surface (32) towards the support rod (1).

21. Posterior intervertebral implant as claimed in claim 20, wherein the embossed surface (32) has a countersink.

22. Posterior intervertebral implant as claimed in claim 22, wherein the countersink is conical.

23. Posterior intervertebral implant as claimed in any one of claims 16 to 22, wherein the stud screw tapers to a point at the bottom.

24. Posterior intervertebral implant as claimed in any one of claims 1 to 23, wherein the fastening member (8) is provided at the bottom with a rotatable spherically shaped contacting element.

25. Posterior intervertebral implant as claimed in claim 24, wherein the rotatable spherically shaped contacting element is configured in the form of a spherical segment (34).

26. Posterior intervertebral implant as claimed in any one of claims 1 to 25, wherein the fixation head (3) is provided with longitudinal serrations (33) on the base of the U-shaped slot (6).

27. Posterior intervertebral implant as claimed in any one of claims 1 to 26, wherein the fixation head (3) is provided with a transverse pattern on the base of the U-shaped slot (6) where the longitudinal rod (1) can sink in.

28. Posterior intervertebral implant as claimed in any one of claims 1 to 27, wherein the base of the U-shaped slot (6) has more than one radius.

29. Posterior intervertebral implant as claimed in claim 28, wherein the base of the U-shaped slot (6) has two radii Rₐ, R_{b}.

30. Posterior intervertebral implant as claimed in any one of claims 1 to 29, wherein the diameter of the fixation head (3) and closure element (7) is in the range of 12 to 14 mm.

31. Posterior intervertebral implant as claimed in claim 30, wherein the diameter of the fixation head (3) and closure element (7) is in the range of 12,5 to 13,5 mm.

32. Posterior intervertebral implant as claimed in any one of claims 1 to 31, wherein the diameter of the fastening element (8) is in the range of 7 to 9 mm.

33. Posterior intervertebral implant as claimed in claim 32, wherein the diameter of the fastening element (8) is in the range of 7,5 to 8,5 mm.

34. Posterior intervertebral implant as claimed in any one of claims 1 to 33, wherein the width of the U-shaped slot (6) in the fixation head (3) and the diameter of the support rod (1) is in the range of 5 to 7 mm.

35. Posterior intervertebral implant as claimed in any one of claims 1 to 34, wherein the width of the U-shaped slot (6) in the fixation head (3) and the diameter of the support rod (1) is in the range of 5,5 to 6,5 mm.

36. Posterior intervertebral implant as claimed in any one of claims 1 to 25 or 30 to 35, wherein the base of the U-shaped slot (6) comprises a concave recess in its longitudinal direction and a movable segment (36) being inserted therein, whereby the movement of the segment (36) is defined by the geometry of the concave recess.

37. Posterior intervertebral implant as claimed in claim 36, wherein the movement of the segment is circular, whereby the centre of rotation is identical with the centre of the spherically shaped contacting element (34).

38. Posterior intervertebral implant as claimed in claim 36, wherein the movement of the segment is circular, whereby the centre of rotation is at the location on the support rod (1) where the tip of the fastening member (8) lies on.

39. Posterior intervertebral implant as claimed in any one of claims 1 to 23, wherein the recess in the base of the U-shaped slot (6) is spherically shaped.

40. Posterior intervertebral implant as claimed in claim 39, wherein the radius of the recess is identical to a ball which is pushed over the support rod (1).

41. Posterior intervertebral implant as claimed in claim 40, wherein the ball being pushed over the support rod (1) is slotted on one side.

42. Posterior intervertebral implant as claimed in any one of claims 1 to 41, wherein the fastening member (8) comprises a flange (37) towards the support rod (1).

43. Posterior intervertebral implant as claimed in claim 42, wherein the flange (37) has an angle α (39) between 0° and 90° on the side opposite to the support rod (1).

44. Posterior intervertebral implant as claimed in claim 43, wherein the flange (37) has an angle α (39) between 45° and 60° on the side opposite to the support rod (1).

45. Posterior intervertebral implant as claimed in any one of claims 42 to 44, wherein the closure element (7) comprises a countersink (38) towards the support rod (1) corresponding to the geometry of the flange (37).

## Revendications

1. Implant vertébral postérieur pour un dispositif présentant une barre d'appui (1) destiné à soutenir la colonne vertébrale, comprenant
A) un segment d'ancrage (2) conçu sous forme de vis ou de crochet et une tête de fixation (3) qui présente une fente en forme de U (6) formant deux branches (4, 5) pour la barre d'appui (1) ; et comprenant
B) des moyens de fixation (7, 8) pour fixer la tête de fixation (3) à la barre d'appui (1), les moyens de fixation (7, 8) étant réalisés en deux parties et présentant une partie de fermeture cylindrique (7) et une partie de fixation (8) ; dans lequel
C) la partie de fermeture cylindrique (7) peut être insérée axialement par le haut dans la fente en forme de U (6) ; et
D) la partie de fixation (8) peut être mise en appui sur la barre d'appui (1) par l'intermédiaire de la première partie de fermeture (7),
**caractérisé en ce que**
E) la partie de fermeture cylindrique (7) et la fente en forme de U (6) sont conçues de telle sorte que la partie de fermeture cylindrique (7) peut être bloquée dans la fente en forme de U (6) sans engendrer de serrage de la barre d'appui en forme de cylindre circulaire (1), de sorte que seule la partie de fixation (8) peut être mise en appui sur la barre d'appui (1).

2. Implant vertébral postérieur selon la revendication 1, **caractérisé en ce que** la partie de fermeture (7) présente un alésage central (10) pourvu d'un taraudage (9) dans lequel la partie de fixation (8) peut être insérée.

3. Implant vertébral postérieur selon la revendication 1, **caractérisé en ce que** la partie de fixation (8) présente un filetage (11) correspondant au taraudage (9) de la partie de fermeture (7).

4. Implant vertébral postérieur selon l'une quelconque des revendications 1 à 3, **caractérisé en ce que** le diamètre extérieur des moyens de fixation (7, 8) est inférieur ou tout au plus égal au diamètre extérieur de la tête de fixation (3).

5. Implant vertébral postérieur selon l'une quelconque des revendications 1 à 4, **caractérisé en ce qu'**une douille de sécurité (12) recouvrant les deux branches (4, 5) de la tête de fixation (3) est prévue.

6. Implant vertébral postérieur selon la revendication 5, **caractérisé en ce que** la douille de sécurité (12) présente un diamètre intérieur si grand qu'elle peut être glissée sur les extrémités des branches (15, 16) effilées vers le haut de la tête de fixation (3).

7. Implant vertébral postérieur selon la revendication 6, **caractérisé en ce que** la douille de sécurité (12) présente sur le haut un collet (13) avec un diamètre intérieur si grand que l'on peut y faire passer la partie de fixation (8).

8. Implant vertébral postérieur selon la revendication 6, **caractérisé en ce que** la douille de sécurité (12) présente sur le haut un plateau avec un alésage si grand que la partie de fixation (8) sous-jacente peut être manipulée avec un instrument correspondant.

9. Implant vertébral postérieur selon l'une quelconque des revendications 5 à 8, **caractérisé en ce que** la douille de sécurité (12) et la partie de fermeture (7) sont assemblées pour former une seule pièce ou sont réalisées en une seule pièce.

10. Implant vertébral postérieur selon l'une quelconque des revendications 1 à 9, **caractérisé en ce que** la partie de fermeture (7) présente un filetage (17) et la fente en forme de U (6) présente un taraudage (18) correspondant à celui-ci.

11. Implant vertébral postérieur selon l'une quelconque des revendications 1 à 9, **caractérisé en ce que** la partie de fermeture (7) présente, sur sa face externe (19), au moins un ergot (20, 21), et les deux branches (4, 5) de la fente en forme de U (6) présentent, sur leurs faces internes (22, 23), des rainures circulaires correspondant à ceux-ci (24, 25), de sorte qu'après l'insertion axiale de la partie de fermeture (7) dans la fente en forme de U (6), on obtient un verrouillage de type à baïonnette (20, 21 ; 24, 25).

12. Implant vertébral postérieur selon la revendication 10, **caractérisé en ce que** la partie de fermeture (7) présente, sur sa face externe (19), deux ergots opposés (20, 21).

13. Implant vertébral postérieur selon l'une quelconque des revendications 1 à 9, **caractérisé en ce que** la partie de fermeture (7) présente, sur sa face externe (19), une rainure transversale circulaire (26) et des rainures longitudinales (27, 28) dont le nombre et la position correspondent, dans lequel
a) la position et le sens des rainures longitudinales (27,28) correspondent aux ergots (29, 30) se trouvant sur les faces internes (22, 23) des deux branches (4, 5) ; et
b) les ergots (29, 30) peuvent être insérés dans la rainure transversale circulaire (26) par un mouvement de rotation après l'insertion axiale de la partie de fermeture (7) dans la fente en forme de U (6).

14. Implant vertébral postérieur selon la revendication 13, **caractérisé en ce que** la partie de fermeture (7) présente, sur sa face externe (19), une rainure transversale circulaire (26) et deux rainures longitudinales opposées (27, 28).

15. Implant vertébral postérieur selon les revendications 11 à 14, **caractérisé en ce que** la rainure transversale circulaire (24, 26) n'occupe pas toute la circonférence de la tête de fixation (3) ou de la partie de fermeture (7).

16. Implant vertébral postérieur selon l'une quelconque des revendications 1 à 15, **caractérisé en ce que** la partie de fixation (8) est conçue sous forme de vis sans tête.

17. Implant vertébral postérieur selon la revendication 16, **caractérisé en ce que** la vis sans tête présente sur le haut une fente (31) et un alésage central (35).

18. Implant vertébral postérieur selon la revendication 17, **caractérisé en ce que** l'alésage central (35) est pourvu d'un filetage.

19. Implant vertébral postérieur selon l'une quelconque des revendications 16 à 18, **caractérisé en ce que** la vis sans tête présente une surface convexe (32) dirigée vers la barre d'appui (1).

20. Implant vertébral postérieur selon la revendication 19, **caractérisé en ce que** la vis sans tête présente une surface bombée (32) dirigée vers la barre d'appui (1).

21. Implant vertébral postérieur selon la revendication 20, **caractérisé en ce que** la surface bombée (32) est pourvu d'un enfoncement.

22. Implant vertébral postérieur selon la revendication 21, **caractérisé en ce que** l'enfoncement a une forme conique.

23. Implant vertébral postérieur selon l'une quelconque des revendications 16 à 22, **caractérisé en ce que** la vis sans tête se termine en bas par une pointe.

24. Implant vertébral postérieur selon l'une quelconque des revendications 1 à 23, **caractérisé en ce que** la partie de fixation (8) est pourvue sur le bas d'un élément de contact sphérique rotatif.

25. Implant vertébral postérieur selon la revendication 24, **caractérisé en ce que** l'élément de contact sphérique rotatif a la forme d'un segment sphérique (34).

26. Implant vertébral postérieur selon l'une quelconque des revendications 1 à 25, **caractérisé en ce que** la tête de fixation (3) est pourvue, au fond de la fente en forme de U (6), de dentures longitudinales (33).

27. Implant vertébral postérieur selon l'une quelconque des revendications 1 à 26, **caractérisé en ce que** la tête de fixation (3) est pourvue, au fond de la fente en forme de U (6), d'une texture transversale dans laquelle la barre d'appui (1) peut se loger.

28. Implant vertébral postérieur selon l'une quelconque des revendications 1 à 27, **caractérisé en ce que** le fond de la fente en forme de U (6) présente plus d'un rayon.

29. Implant vertébral postérieur selon la revendication 28, **caractérisé en ce que** le fond de la fente en forme de U (6) présente deux rayons Rₐ, R_{b}.

30. Implant vertébral postérieur selon l'une quelconque des revendications 1 à 29, **caractérisé en ce que** le diamètre de la tête de fixation (3) et de l'élément de blocage (7) est dans la gamme de 12 à 14 mm.

31. Implant vertébral postérieur selon la revendication 30, **caractérisé en ce que** le diamètre de la tête de fixation (3) et de l'élément de blocage (7) est dans la gamme de 12,5 à 13,5 mm.

32. Implant vertébral postérieur selon l'une quelconque des revendications 1 à 31, **caractérisé en ce que** le diamètre de la partie de fixation (8) est dans la gamme de 7 à 9 mm.

33. Implant vertébral postérieur selon la revendication 32, **caractérisé en ce que** le diamètre de la partie de fixation (8) est dans la gamme de 7,5 à 8,5 mm.

34. Implant vertébral postérieur selon l'une quelconque des revendications 1 à 33, **caractérisé en ce que** la largeur de la fente en forme de U (6) dans la tête de fixation (3) et le diamètre de la barre d'appui (1) sont dans la gamme de 5 à 7 mm.

35. Implant vertébral postérieur selon l'une quelconque des revendications 1 à 34, **caractérisé en ce que** la largeur de la fente en forme de U (6) dans la tête de fixation (3) et le diamètre de la barre d'appui (1) sont dans la gamme de 5,5 à 6,5 mm.

36. Implant vertébral postérieur selon l'une quelconque des revendications 1 à 25 ou 30 à 35, **caractérisé en ce que** le fond de la fente en forme de U (6) présente, sur la longueur, un évidement concave, et un segment mobile (36) y est inséré, le mouvement du segment (36) étant défini par la géométrie de l'évidement concave.

37. Implant vertébral postérieur selon la revendication 36, **caractérisé en ce que** le mouvement du segment est circulaire, le centre de rotation étant identique au centre de l'élément de contact sphérique (34).

38. Implant vertébral postérieur selon la revendication 36, **caractérisé en ce que** le mouvement du segment est circulaire, le centre de rotation étant situé à l'endroit de la barre d'appui (1) où la pointe de la partie de fixation (8) prend appui.

39. Implant vertébral postérieur selon l'une quelconque des revendications 1 à 23, **caractérisé en ce que** l'évidement au fond de la fente en forme de U (6) a une forme sphérique.

40. Implant vertébral postérieur selon la revendication 39, **caractérisé en ce que** le rayon de l'évidement est identique à celui d'une sphère glissée sur la barre d'appui (1).

41. Implant vertébral postérieur selon la revendication 40, **caractérisé en ce que** la sphère glissée sur la barre d'appui (1) est fendue sur un côté.

42. Implant vertébral postérieur selon l'une quelconque des revendications 1 à 41, **caractérisé en ce que** la partie de fixation (8) présente une collerette (37) dirigée vers la barre d'appui (1).

43. Implant vertébral postérieur selon la revendication 42, **caractérisé en ce que,** sur le côté opposé à la barre d'appui (1), la collerette (37) présente un angle α (39) compris entre 0° et 90°.

44. Implant vertébral postérieur selon la revendication 43, **caractérisé en ce que,** sur le côté opposé à la barre d'appui (1), la collerette (37) présente un angle α (39) compris entre 45° et 60°.

45. Implant vertébral postérieur selon l'une quelconque des revendications 42 à 44, **caractérisé en ce que** la partie de fermeture (7) présente un chanfrein (38) dirigé vers la barre d'appui (1), correspondant à la géométrie de la collerette (37).
